# EUROPEAN PATENT SPECIFICATION

(11) **EP 4 386 452 B1**
(45) Date of publication and mention of the grant of the patent: **14.10.2026**
(21) Application number: 21953852.7
(22) Date of filing: 24.08.2021
(51) Int. Cl.: G01V 8/12, G01N 15/01, G01N 15/1434, G01N 15/14, A61M 1/36

(54) **HIGH-RELIABILITY CELL DETECTOR**
ZELLDETEKTOR MIT HOHER ZUVERLÄSSIGKEIT
DÉTECTEUR DE CELLULES À HAUTE FIABILITÉ

(30) Priority: 16.08.2021 CN 202110941067
(43) Date of publication of application: 19.06.2024
(73) Proprietor: Scinomed Limited, Harrogate, England HG1 2PW (GB)
(72) Inventor: SONG, HongBiao, Shanghai 200126 (CN); WANG, Xiao, Shanghai 201206 (CN)
(74) Representative: Mohun, Stephen John
(86) International application number: PCT/CN2021/114287
(87) International publication number: WO 2023/019611

(56) References cited:
- EP-B1- 1 144 026
- CN-A- 106 267 426
- CN-A- 107 167 216
- CN-A- 107 782 702
- CN-A- 107 790 298
- CN-U- 204 286 544
- CN-U- 204 374 094
- CN-U- 205 138 590
- CN-U- 205 449 628
- CN-U- 206 228 671
- CN-U- 207 423 630
- CN-U- 212 569 969
- CN-U- 213 398 602
- KR-A- 20030 061 753
- US-A- 4 227 814
- US-A1- 2005 054 508
- US-A1- 2006 155 236
- US-A1- 2018 128 730
- US-A1- 2020 261 630
- US-B1- 6 174 447

## Description

### Technical Field

The present invention belongs to the technical field of auxiliary devices for blood plasma collection machines, specifically relating to a high-reliability cell detector.

### Background Art

A blood plasma collection machine is a machine that uses the principle of centrifugation to collect blood plasma. It mainly consists of a centrifuge, display, machine body, liquid pump, and other components. It can quickly complete the collection of blood plasma. It can be used not only for the collection of raw material blood plasma for the production of blood products by blood product companies but also for the collection of fresh frozen plasma, with broad practicality.

Existing blood plasma collection machines, when in use, transport external blood to the inside of the plasma collection machine through a transfusion tube. Existing plasma collection machines do not have the function to detect blood within the transfusion tube, which can inconvenience operators in determining whether there is blood in the transfusion tube. Therefore, this disclosure proposes a high-reliability cell detector.

EP 1 144 026 B1 describes a system for separating biological fluids into components, the system comprising a set of containers (40, 42-44) for the biological fluid to be separated and the separated components, optionally an additional container (41) for additive solution, and a hollow centrifugal processing chamber (20) having an axial inlet/outlet (7) for the biological fluid.

US 6 174 447 B1 discloses a method for fluid separation of whole blood as a mixture of liquids into individual, differently-colored blood constituents, which blood is packed in flexible containers, in particular bags, wherein the bags are connected to one another with an at least partially light-transparent connection, in particular a flexible tube, and wherein the blood constituents are forced to flow from one container through the light-transparent connection into another container, in particular for the separation of concentrated thrombocytes from buffy coat, as well as to a device for performing the method.

US 2020/261630 A1 describes a method for collecting plasma including determining the weight and hematocrit of a donor, and inserting a venous-access device into the donor. The method then withdraws blood from the donor through a draw line connected to a blood component separation device, and introduces anticoagulant into the withdrawn blood. The blood component separation device separates the blood into a plasma component and a second blood component, and the plasma component is collected from the blood component separation device and into a plasma collection container.

US 2006/155236 A1 discloses methods and apparatus for collecting and treating blood components. A cassette having flexible tubes inside its housing and on the bottom surface outside the cassette housing for ease of manufacturing is operated with a centrifuge bowl having an inlet and two outlets, pressure domes for measuring pressures in tubes, an improved irradiation plate, and a dual chamber bag in an extracorporeal photopheresis treatment.

US2005/054508 A1 discloses a control device for the separate collection of blood components in output from a blood centrifugation cell. The control device comprises: a support which comprises a recess for containing a duct; a visible-light emitter with a corresponding receiver, which are arranged on opposite sides with respect to the recess for containing the duct; an infrared-light emitter with a corresponding receiver, which are arranged on opposite sides with respect to the recess for containing the duct; and a processor to detect signals acquired by the two receivers, to calculate a ratio between the values of the signals, and to send signals to the means suitable to selectively send the fluid conveyed by the duct into separate collection bags, the means being able to change the bag in which the fluid is collected.

US 4 227 814 A describes an optical detector that is particularly adapted for use with an apparatus for separating blood into its components and is of the type including a centrifuge for centrifuging whole blood to separate same into the components thereof and of the type wherein plasma is withdrawn from the centrifuge through a flexible, light transmitting tubing and the spillover of red blood cells into the plasma withdrawn is monitored by the optical detector.

### Summarv of the Invention

The purpose of this disclosure is to provide a high-reliability cell detector to solve the problems mentioned in the background technology.

The invention is defined in the attached independent claim. Preferred features are found in the dependent claims appended thereto.

To achieve the above objectives, the present disclosure provides the following technical solutions: a high-reliability cell detector, including a machine body and a blood cell detector installed on the top of the machine body. The blood cell detector includes a vascular frame installed on the top surface of the machine body, with a U-shaped tube orifice at the top of the vascular frame. The inner wall of the U-shaped tube orifice is embedded with a transmissive photo sensor, which includes a light emitter and a light receiver, and the light emitter and light receiver are respectively embedded in the inner walls on both sides of the U-shaped tube orifice.

Preferably, the bottom surface of the vascular frame is provided with a circular mounting groove, and an electromagnetic suction cup is embedded within the circular mounting groove. The surface of the electromagnetic suction cup is equipped with a connecting wire, which passes to the outside of the vascular frame. The electromagnetic suction cup is magnetically connected to the top surface of the machine body.

Preferably, L-shaped side slots are symmetrically provided on both side surfaces of the vascular frame. An L-shaped block is arranged inside the L-shaped side slot, and a limit card head is fixed at the bottom end of the L-shaped block. Limit card slots are provided on both side surfaces of the electromagnetic suction cup, and the end of the limit card head penetrates to the inside of the limit card slot.

Preferably, a rectangular slot is provided on one inner wall of the L-shaped side slot, and a spring and a side slider are arranged inside the rectangular slot. The side slider is fixed to the top end of the L-shaped block.

Preferably, a protruding rod is fixed to one side surface of the L-shaped block, and a rotating pull block is rotatably mounted at the end of the protruding rod.

Preferably, base pads are installed on the inner walls on both sides of the U-shaped tube orifice, opposite the top of the transmissive photo sensor, and the surface of the base pad is provided with strip protrusions.

Preferably, the base pad and the strip protrusion are of an integrated structure, and the cross-section of the strip protrusion is semi-circular.

Compared with the existing technology, the beneficial effects of the disclosure are:
1. By installing a blood cell detector on the top of the blood plasma collection machine, the transfusion tube can be clamped at the top of the blood cell detector, allowing the blood cell detector to detect in real-time whether there are blood cells in the transfusion tube. The blood cell detector has high recognition accuracy, high reliability, and strong resistance to bubble interference.
2. By embedding an electromagnetic suction cup at the bottom of the blood cell detector, the installation position of the blood cell detector can be flexibly adjusted according to actual usage, greatly improving the flexibility of the blood cell detector, and the electromagnetic suction cup is easy to install and remove.
3. By setting the base pad and strip protrusion, a good limiting effect can be achieved for the transfusion tube entering the inside of the U-shaped tube orifice, thus improving the stability of the use of the blood cell detector.

### Brief Description of the Drawings

FIG. 1 is a perspective view of the disclosure installed on a blood plasma collection machine;
FIG. 2 is a sectional view of the disclosure;
FIG. 3 is a partial enlarged view of area A in FIG. 2;
FIG. 4 is a partial enlarged view of area B in FIG. 2;

List of reference numerals: 1 - vascular frame; 2 - U-shaped tube orifice; 3 - transmissive photo sensor; 4 - machine body; 5 - electromagnetic suction cup; 6 - L-shaped side slot; 7 - L-shaped block; 8 - rotating pull block; 9 - rectangular slot; 10 - spring; 11 - side slider; 12 - limit card head; 13 - limit card slot; 14 - base pad; 15 - strip protrusion.

### Detailed Description of Embodiments

The technical solutions in the embodiments of the present disclosure will be clearly and completely described below in conjunction with the accompanying drawings in the embodiments of the present disclosure. It is obvious that the described embodiments are only part of the embodiments of the present disclosure, not all embodiments. Based on the embodiments in this disclosure, all other embodiments obtained by those of ordinary skill in the art without creative efforts fall within the protection scope of the present disclosure.

### Embodiment

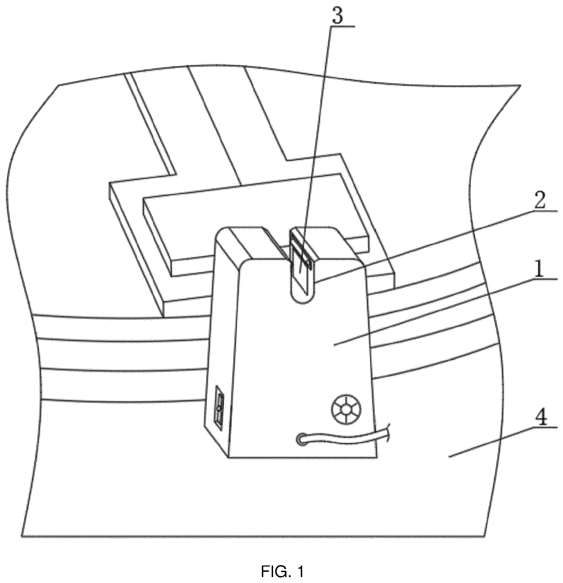

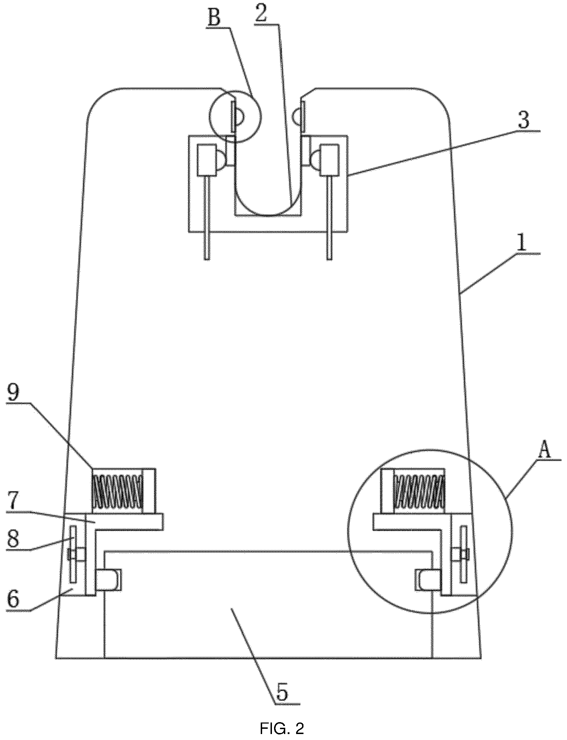

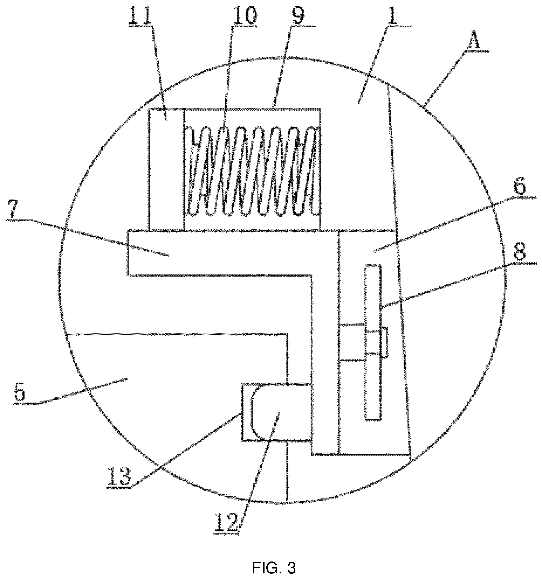

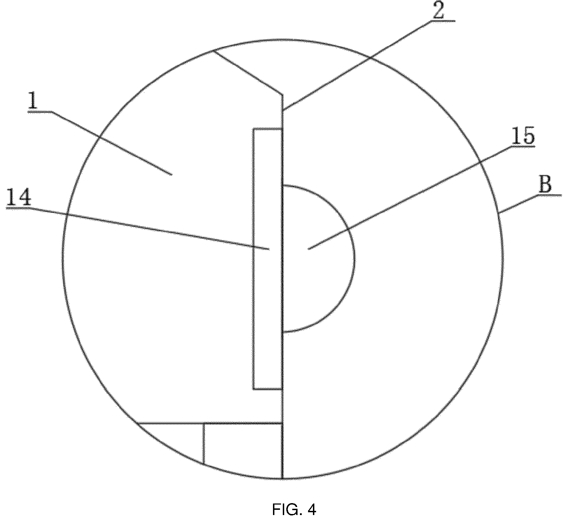

Please refer to FIGS. 1 to 4, the disclosure provides a technical solution: a high-reliability cell detector, including a machine body 4 and a blood cell detector installed on the top of the machine body 4. The blood cell detector includes a vascular frame 1 installed on the top surface of the machine body 4, with a U-shaped tube orifice 2 at the top of the vascular frame 1, and the inner wall of the U-shaped tube orifice 2 is embedded with a transmissive photo sensor 3. The transmissive photo sensor 3 includes a light emitter and a light receiver, and the light emitter and light receiver are respectively embedded in the inner walls on both sides of the U-shaped tube orifice 2, allowing the transmissive photo sensor 3 to detect in real-time whether there are blood cells inside the transfusion tube through the light emitter and light receiver. The surface of the vascular frame 1 is provided with an adjustment hole below and to the right of the U-shaped tube orifice 2, and an adjustment bolt is set inside the adjustment hole. The inside of the vascular frame 1 is equipped with a PLC controller connected to the adjustment bolt, allowing the operator to later insert a screwdriver into the adjustment hole and then twist the adjustment bolt, causing the adjustment bolt to adjust the detection sensitivity of the cell detector through the PLC controller.

In this embodiment, preferably, the bottom surface of the vascular frame 1 is provided with a circular mounting groove, and an electromagnetic suction cup 5 is embedded within the circular mounting groove. The surface of the electromagnetic suction cup 5 is equipped with a connecting wire, which passes to the outside of the vascular frame 1. The electromagnetic suction cup 5 is magnetically connected to the top surface of the machine body 4, and the magnetic installation method allows the installation position of the blood cell detector to be flexibly adjusted according to actual usage.

In this embodiment, preferably, L-shaped side slots 6 are symmetrically provided on both side surfaces of the vascular frame 1. An L-shaped block 7 is arranged inside the L-shaped side slot 6, and a limit card head 12 is fixed at the bottom end of the L-shaped block 7. Limit card slots 13 are provided on both side surfaces of the electromagnetic suction cup 5, and the end of the limit card head 12 penetrates to the inside of the limit card slot 13. A rectangular slot 9 is provided on one inner wall of the L-shaped side slot 6, and a spring 10 and a side slider 11 are arranged inside the rectangular slot 9. The side slider 11 is fixed to the top end of the L-shaped block 7. A protruding rod is fixed to one side surface of the L-shaped block 7, and a rotating pull block 8 is rotatably mounted at the end of the protruding rod, making the electromagnetic suction cup 5 easy to install and remove.

In this embodiment, preferably, base pads 14 are adhesively installed on the inner walls on both sides of the U-shaped tube orifice 2, opposite the top of the transmissive photo sensor 3, and the surface of the base pad 14 is provided with strip protrusions 15. The base pad 14 and the strip protrusion 15 are of an integrated structure, and both the base pad 14 and the strip protrusion 15 are made of rubber material. The cross-section of the strip protrusion 15 is semi-circular, which can provide a good limiting effect for the transfusion tube entering the inside of the U-shaped tube orifice 2.

In this disclosure, the model of the transmissive photo sensor 3 is Omron EE-SX1350 transmissive;

In this disclosure, the model of the electromagnetic suction cup 5 is KTF-05;

The working principle and usage process of the disclosure: When using the blood cell detector, first install the vascular frame 1 on the top of the machine body 4, then push the transfusion tube that transports blood into the inside of the U-shaped tube orifice 2, and ensure that the transfusion tube is limited by the strip protrusion 15, allowing the transmissive photo sensor 3 to detect in real-time whether there are blood cells inside the transfusion tube through the light emitter and light receiver;
When it is necessary to install the electromagnetic suction cup 5 at the bottom of the vascular frame 1, first pull the rotating pull block 8 to the side, causing the L-shaped block 7 and limit card head 12 to move to the side, until the rotating pull block 8 is pulled to the outside of the vascular frame 1. Then, rotate the rotating pull block 8 ninety degrees, allowing the ends of the rotating pull block 8 to rest on the outer surface of the vascular frame 1, preventing the L-shaped block 7 and limit card head 12 from rebounding. Next, push the electromagnetic suction cup 5 into the circular mounting groove at the bottom of the vascular frame 1, then rotate the rotating pull block 8 back and release it, causing the spring 10 to push back the side slider 11, thereby causing the L-shaped block 7 and limit card head 12 to rebound, and finally allowing the limit card head 12 to snap into the limit card slot 13 on the surface of the electromagnetic suction cup 5, completing the quick installation of the electromagnetic suction cup 5. Then, power on the electromagnetic suction cup 5 to cause it to adhere tightly to the top surface of the machine body 4, completing the quick installation of the blood cell detector. To adjust the position of the blood cell detector subsequently, simply power off the electromagnetic suction cup 5, adjust the position of the blood cell detector, and then power on the electromagnetic suction cup 5 again to complete the magnetic positioning of the vascular frame 1 once more, allowing the installation position of the blood cell detector to be flexibly adjusted according to actual usage.

## Claims

1. A high-reliability cell detector including a machine body (4) and a blood
cell detector mounted on a top of the machine body (4), the blood cell detector comprises a vascular frame (1) mounted on a top surface of the machine body (4), a top end of the vascular frame (1) is provided with a U-shaped tube orifice (2), and an inner wall of the U- shaped tube orifice (2) is embedded with a transmissive photo sensor (3), the transmissive photo sensor (3) includes a light emitter and a light receiver, and the light emitter and light
receiver are respectively embedded on two side inner walls of the U-shaped tube orifice (2)
**characterized in that**: a bottom surface of the vascular frame (1) is provided with a circular mounting groove, and an electromagnetic suction cup (5) is embedded in the circular mounting groove, the surface of the electromagnetic suction cup (5) is provided with a connecting wire, the connecting wire runs through to the outside of the vascular frame (1), and the electromagnetic suction cup (5) is magnetically connected to the top surface of the machine body (4).

2. The high-reliability cell detector according to Claim 1, **characterized in that**: two side surfaces of the vascular frame (1) are symmetrically provided with L-shaped side slots (6), an inside of the L-shaped side slot (6) is provided with an L-shaped block (7), a bottom end of the L-shaped block (7) is fixed with a limit card head (12), two side surfaces of the electromagnetic suction cup (5) are provided with limit card slots (13), and an end of the limit card head (12) runs through to the inside of the limit card slot (13).

3. The high-reliability cell detector according to Claim 2, **characterized in that**: one side inner wall
of the L-shaped side slot (6) is provided with a rectangular slot (9), and the rectangular slot (9) is provided with a spring (10) and a side slider (11), the side slider (11) is fixed to the top end of the L-shaped block (7).

4. The high-reliability cell detector according to Claim 2, **characterized in that**: one side surface of the L-shaped block (7) is fixed with a protruding rod, and the end of the protruding rod is rotatably provided with a rotating pull block (8).

5. The high-reliability cell detector according to Claim 1, **characterized in that**: the two side inner walls of the U-shaped tube orifice (2) are provided with a base pad (14) opposite the top of the transmissive photo sensor (3), and a surface of the base pad (14) is provided with a strip protrusion (15).

6. The high-reliability cell detector according to Claim 5, **characterized in that**: the base pad (14) and the strip protrusion (15) are of an integral structure, and a cross-section of the strip protrusion (15) is semi-circular.

## Patentansprüche

1. Zelldetektor mit hoher Zuverlässigkeit, beinhaltend einen Maschinenkörper (4) und einen Blutzellendetektor, der an einer Oberseite des Maschinenkörpers (4) montiert ist, wobei der Blutzellendetektor einen Gefäßrahmen (1) umfasst, der an einer oberen Oberfläche des Maschinenkörpers (4) montiert ist, wobei ein oberes Ende des Gefäßrahmens (1) mit einer U-förmigen Rohröffnung (2) versehen ist und eine Innenwand der U-förmigen Rohröffnung (2) mit einem transmissiven Fotosensor (3) eingebettet ist, wobei der transmissive Fotosensor (3) einen Lichtsender und einen Lichtempfänger beinhaltet und der Lichtsender und der Licht-
empfänger jeweils an zwei seitlichen Innenwänden der U-förmigen Rohröffnung (2) eingebettet sind,
**dadurch gekennzeichnet, dass**: eine untere Oberfläche des Gefäßrahmens (1) mit einer kreisförmigen Montagenut versehen ist und ein elektromagnetischer Saugnapf (5) in die kreisförmige Montagenut eingebettet ist, die Oberfläche des elektromagnetischen
Saugnapfs (5) mit einem Anschlussdraht versehen ist, der Anschlussdraht zur Außenseite des Gefäßrahmens (1) verläuft und der elektromagnetische Saugnapf (5) magnetisch mit der oberen Oberfläche des Maschinenkörpers (4) verbunden ist.

2. Zelldetektor mit hoher Zuverlässigkeit nach Anspruch 1, **dadurch gekennzeichnet, dass**: zwei Seitenoberflächen des Gefäßrahmens (1) symmetrisch mit L-förmigen Seitenschlitzen (6) versehen sind, eine Innenseite des L-förmigen Seitenschlitzes (6) mit einem L-förmigen Block (7) versehen ist, ein unteres Ende des L-förmigen Blocks (7) mit einem Begrenzungskartenkopf (12) fixiert ist, zwei Seitenoberflächen des elektromagnetischen Saugnapfs (5) mit Begrenzungskartenschlitzen (13) versehen sind und ein Ende des Begrenzungskartenkopfes (12) zur Innenseite des Begrenzungskartenschlitzes (13) verläuft.

3. Zelldetektor mit hoher Zuverlässigkeit nach Anspruch 2, **dadurch gekennzeichnet, dass**: eine seitliche Innenwand des L-förmigen Seitenschlitzes (6) mit einem rechteckigen Schlitz (9) versehen ist und der rechteckige Schlitz (9) mit einer Feder (10) und einem Seitengleiter (11) versehen ist, wobei der Seitengleiter (11) am oberen Ende des L-förmigen Blocks (7) fixiert ist.

4. Zelldetektor mit hoher Zuverlässigkeit nach Anspruch 2, **dadurch gekennzeichnet, dass**: eine Seitenoberfläche des L-förmigen Blocks (7) mit einer vorstehenden Stange fixiert ist und das Ende der vorstehenden Stange drehbar mit einem rotierenden Zugblock (8) versehen ist.

5. Zelldetektor mit hoher Zuverlässigkeit nach Anspruch 1, **dadurch gekennzeichnet, dass**: die zwei seitlichen Innenwände der U-förmigen Rohröffnung (2) mit einer Basisauflage (14) gegenüber der Oberseite des transmissiven Fotosensors (3) versehen sind und eine Oberfläche der Basisauflage (14) mit einem Streifenvorsprung (15) versehen ist.

6. Zelldetektor mit hoher Zuverlässigkeit nach Anspruch 5, **dadurch gekennzeichnet, dass**: die Basisauflage (14) und der Streifenvorsprung (15) von integraler Struktur sind und ein Querschnitt des Streifenvorsprungs (15) halbkreisförmig ist.

## Revendications

1. Détecteur de cellules à haute fiabilité comprenant un corps de machine (4) et un détecteur de cellules sanguines monté sur le haut du corps de machine (4), le détecteur de cellules sanguines comprend un cadre vasculaire (1) monté sur une surface supérieure du corps de machine (4), une extrémité supérieure du cadre vasculaire (1) est munie d'un orifice de tube en forme de U (2), et une paroi interne de l'orifice de tube en forme de U (2) est encastrée avec un capteur photo transmissif (3), le capteur photo transmissif (3) comprend un émetteur de lumière et un récepteur de lumière, et l'émetteur de lumière et le récepteur de lumière sont respectivement encastrés sur deux parois internes latérales de l'orifice de tube en forme de U (2)
**caractérisé en ce que** : une surface inférieure du cadre vasculaire (1) est pourvue d'une rainure de montage circulaire, et une ventouse électromagnétique (5) est encastrée dans la rainure de montage circulaire, la surface de la ventouse électromagnétique (5) est pourvue d'un fil de connexion, le fil de connexion s'étend jusqu'à l'extérieur du cadre vasculaire (1), et la ventouse électromagnétique (5) est connectée magnétiquement à la surface supérieure du corps de machine (4).

2. Détecteur de cellules à haute fiabilité selon la revendication 1, **caractérisé en ce que** : deux surfaces latérales du cadre vasculaire (1) sont pourvues symétriquement de fentes latérales en forme de L (6), un intérieur de la fente latérale en forme de L (6) est pourvu d'un bloc en forme de L (7), une extrémité inférieure du bloc en forme de L (7) est fixée à une tête de carte de retenue (12), deux surfaces latérales de la ventouse électromagnétique (5) sont pourvues de fentes de carte de retenue (13), et une extrémité de la tête de carte de retenue (12) s'étend jusqu'à l'intérieur de la fente de carte de retenue (13).

3. Détecteur de cellules à haute fiabilité selon la revendication 2, **caractérisé en ce que** : une paroi interne latérale de la fente latérale en forme de L (6) est pourvue d'une fente rectangulaire (9), et la fente rectangulaire (9) est pourvue d'un ressort (10) et d'un coulisseau latéral (11), le coulisseau latéral (11) est fixé à l'extrémité supérieure du bloc en forme de L (7).

4. Détecteur de cellules à haute fiabilité selon la revendication 2, **caractérisé en ce que** : une surface latérale du bloc en forme de L (7) est fixée à une tige en saillie, et l'extrémité de la tige en saillie est pourvue de manière rotative d'un bloc de traction rotatif (8).

5. Détecteur de cellules à haute fiabilité selon la revendication 1, **caractérisé en ce que** : les deux parois internes latérales de l'orifice de tube en forme de U (2) sont pourvues d'un patin de base (14) à l'opposé du haut du capteur photo transmissif (3), et une surface du patin de base (14) est pourvue d'une saillie en bande (15).

6. Détecteur de cellules à haute fiabilité selon la revendication 5, **caractérisé en ce que** : le patin de base (14) et la saillie en bande (15) sont d'une structure solidaire, et une section transversale de la saillie en bande (15) est semi-circulaire.
